# EUROPEAN PATENT APPLICATION

(11) **EP 0 765 727 A2**
(43) Date of publication of application: **02.04.1997**
(21) Application number: 96202644.9
(22) Date of filing: 25.09.1996
(51) Int. Cl.: B29C 45/14, A61M 5/31, B01D 29/00

(54) **Improved filter assembly and method of making same**

(30) Priority: 27.09.1995 US 534879
(71) Applicant: Arbor Technologies, Inc., Ann Arbor, Michigan 48108 (US)
(72) Inventor: Vincent, Monty, Ann Arbor, Michigan 48103 (US); Knoedler, Leonard, Ann Arbor, Michigan 48105 (US)
(74) Representative: Wharton, Peter Robert

(57) **Abstract**

A method of making a filter assembly (10,10') is disclosed. The method includes injection molding a housing member (40,40') having an outlet end (44,44') and an inlet end (42,42') while simultaneously sealing the outlet end (44,44') of the housing (40,40') to an open end (54,54') of a tubular filter media (50,50'). The tubular filter media (50,50') further includes a closed end (52,52') which extends into the housing (40,40'). A filter assembly (10,10') is also disclosed. The assembly (10,10') includes a housing (40,40') having an inlet end (42,42') and an outlet end (44,44') and an internal passageway (46,46') therethrough for allowing fluid flow. The outlet end (44,44') of the housing (40,40') has a smaller diameter than the remainder of the passageway (46,46'). The assembly (10,10') further includes a filter media (48,48') defining a tube (50,50') having a closed end (52,52') and an open end (54,54') wherein the open end (54,54') is sealed to the outlet end (44,44') of the housing (40,40') and the remainder of the tube (50,50') extends into the passageway (46,46').

## Description

### TECHNICAL FIELD

The present invention relates to filter assemblies. More specifically, the present invention relates to a one step method for forming a filter assembly and the filter assembly made thereby.

### BACKGROUND OF THE INVENTION

The use of filtration devices has become more and more predominant in the medical field due to the desire to remove impurities or particulates from fluids, such as injectables, or the like, prior to their administration to a patient. The art is well developed regarding devices which filter out impurities or particulates from fluids.

In particular, microfilters which are adapted for filtration of very small amounts of fluid are known in the art. Microfiltration devices for use in combination with a syringe, such as that shown in United States Patent No.4,453,927 to Sinko, teach a microfiltration device for use on a syringe which includes a multi-piece housing having a flat filter screen disposed therein. This type of microfiltering device has multiple components, is complicated and, therefore, very costly to produce. Specifically, the production of the device requires separate molding of the housing halves then further steps for placement and molding of the screen in place between the halves.

United States Patent Number 4,547,190 to Leason discloses a blood filtering assembly which includes a tubular filter member disposed within a filter chamber and bonded within an inlet opening of the filter chamber through the use of heat or radio waves. The filter assembly is constructed through the use of injection blow molding to form an integral unit. Since the filter is bonded within the opening of the inlet of the filter chamber through the use of heat or radio waves, it requires additional steps during the formation of the filter assembly thereby adding to the complexity and cost of producing the filter assembly.

Therefore, it would be desirable to have a filter assembly for use in filtration operations in which the filter assembly can be produced in a one step process thereby eliminating the complexity and lowering the cost of assembly and manufacturer as set forth in the prior art.

### SUMMARY OF THE INVENTION AND ADVANTAGES

A method according to the present invention of making a filter assembly by injection molding a housing member having an outlet end and an inlet end while simultaneously sealing the outlet end of the housing to an open end of a tubular filter media, the tubular filter media having a closed end extending into the housing.

A filter assembly according to the present invention includes a housing having an inlet end and an outlet end and an internal passageway therethrough for allowing fluid flow, the outlet end being smaller in diameter than the remainder of the passageway. The filter assembly further includes a filter media defining a tube having a closed end and an open end wherein the open end is sealed to the outlet end and the remainder of the tube extends into the passageway.

### BRIEF DESCRIPTION OF THE DRAWINGS

Other advantages of the present invention will be readily appreciated as the same becomes better understood by reference to the following detailed description when considered in connection with the accompanying drawings wherein:
Figure 1 is a perspective view of a syringe and a filter assembly in accordance with the present invention;
Figure 2 is a cross-sectional view of Figure 1 taken along line 2-2;
Figure 3 is an enlarged view of the portion of Figure 2 within circle 3; and
Figure 4 is a cross-sectional view of an alternative embodiment of the present invention.

### DETAILED DESCRIPTION OF THE INVENTION

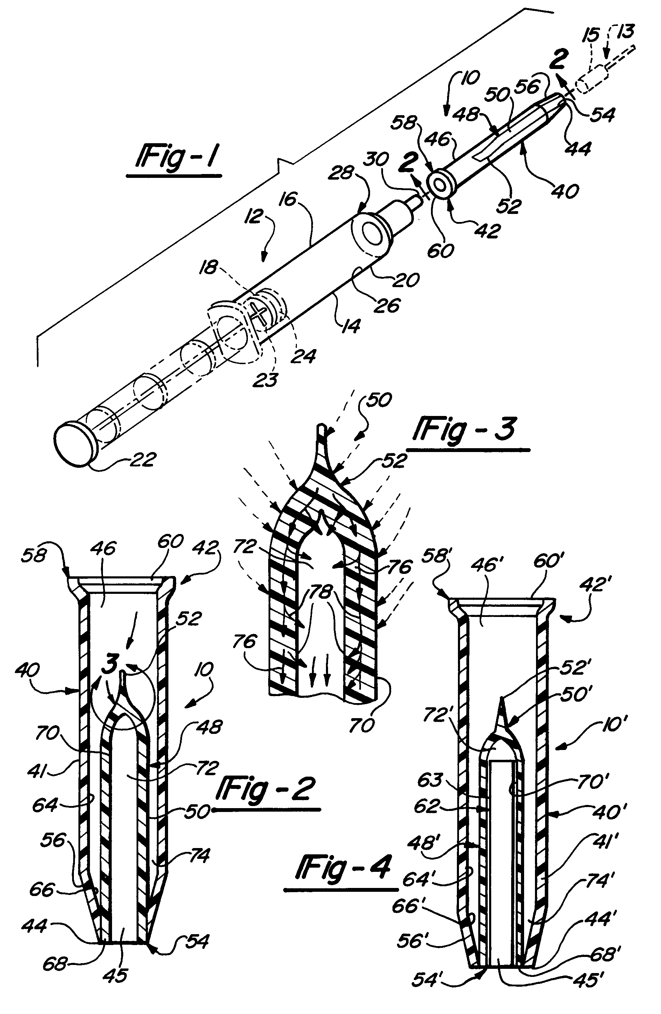

Referring to the Figures, a filter assembly is generally shown and designated by the reference numeral 10. Referring specifically to Figure 1, the filter assembly 10 is shown associated with a syringe 12. The syringe 12 is a conventional syringe and includes a hollow, tubular barrel 14 defining a cavity 16 and being open at its distal end 18 for receiving a plunger 22 adapted for slidable movement therein. A seal or O-ring 24 can be provided on an end 23 of the plunger 22 that is inserted into the cavity 16 of the tubular barrel 14 to create a seal between the plunger 22 and the interior 26 of the cavity 16 to prevent the leakage of solution placed into the cavity 16. The forward end 20 of the syringe barrel 14 is provided with a connector 28 having an opening 30 therethrough for drawing in or releasing fluids to be filtered into or out of the syringe cavity 16.

The filter assembly 10 includes an elongated, single piece hollow tubular housing member 40 manufactured from suitable medical plastic materials, for example polypropylenes, polyolefins, polyesters, polyamides, polycarbonates, polystyrenes, styrenes, co-polymers, and fluoroplastics. The list is not meant to be exhaustive and can include other suitable materials without departing from the present invention.

The tubular housing member 40 includes a cylindrical side wall 41. The housing member 40 further includes an inlet end 42 and an outlet end 44 and an internal passageway 46 extends therebetween for allowing fluid to flow therethrough. An opening 45 is provided in the outlet end 44 which allows filtered fluids to exit the housing member 40.

The filter assembly 10 further includes a filter media 48 in the form of a tubular filter 50 having a closed end 52 and an opened end 54. The open end 54 is sealed to the outlet end 44 of the filter assembly 10 and the remainder of the tubular filter 50 extends into the passageway 46.

The tubular filter 50 projects into the passageway 46 of the housing member 40 at least one quarter to three quarters of the distance between the outlet end 44 and the inlet end 42.

The filter media 48 can be any suitable hydrophobic or hydrophilic material such as cellulose fiber, polysulfones, polyamides, polyolefins, polyesters, and fluoropolymers. This list of materials is not intended to be exhaustive and other suitable materials can be utilized without departing from the present invention.

The housing member 40 includes an external surface 56 which can be tapered. The tapered external surface 56 narrows towards the outlet end 44 of the filter assembly 10. The taper on the external surface 56 of the housing member 40 allows for the hub 15 of a needle 13 (illustrated in phantom) or the like to be fixedly attached to the outlet end 44 of the filter assembly 10.

A connector 58 disposed on the inlet end 42 of the filter assembly 10 allows for connection of the filter assembly 10 to a fluid source such as a syringe 12. The fluid source can also include other suitable fluid sources such as application of the assembly 10 in an in-line application in a sensing device monitoring pressures wherein gases are allowed to pass through filter assembly 10 and the gas pressure is then measured. The connector 58 can include a flange 60 which acts as a male member of a male/female connector. The flange 60 can be inserted into a female member disposed on the forward end 20 of the syringe barrel 14. For example, a typical connector 28 can be a male/female interlocking connector 28 of the Luer-type. Alternatively, the connector 28 can be of the friction fit type.

The inner or internal surface 64 of the housing 40 can be tapered. The tapered internal surface 64 narrows towards the outlet end 44 of the housing member 40. The tapered internal surface 64 directs or guides the flow of liquids entering the housing member 40 and terminates at or near the opening 45 disposed at the outlet end 44 of the housing member 40. The taper also allows for the single steps production of the housing 40, as described below in detail.

The open end 54 of the tubular filter 50 is sealed to an inner surface 66 of the housing member 40 located at the outlet end 44. That is, a peripheral edge 68 of the tubular filter 50 is molded to the internal surface 66 of the housing member 40 at the outlet end 44 such that fluid having already passed through the filter media 48 can exit through the opening 45 in the outlet end 44 of the filter assembly 10. A fluid-tight seal between the tubular filter 50 and the housing member 50 is created by molding the peripheral edge 68 of the open end 54 of the tubular filter 50 to the internal surface 66 of the outlet end 44 of the housing member 40.

Referring to Figures 2 and 3, the tubular filter 5 is generally a hollow tubular member having an elongated cylindrical side wall 70 and a closed or sealed end 52 extending into the passageway 46. The tubular filter 50 defines a cavity 72 therein to enable fluid from the fluid source, such as a syringe 12, to pass out of the tubular filter 5 and exit through the outlet end 44 after filtering. The tubular filter 50 and the cylindrical wall 41 of the housing member 40 define an interstitial space 74 between them. That is, the tubular filter 50 and the side wall 41 of the housing member 40 are disposed in spaced apart relationship enabling fluid from the source and entering the housing 40 to pass into and through the tubular filter 50 along the entire length of the filter 50. The tubular filter 50 is disposed within the concentrically side wall 41 of the housing member 40 thereby creating space for fluid to flow or accumulate prior to filtering.

The fluids filtered by the filter assembly 10 can be both liquids, gases, or both in combination. The assembly 10 can be constructed to filter or separate gases from liquids, liquids from gases, and/or particulates from liquids.

Referring specifically to Figure 3, as fluid is drawn from the outside of the tubular filter 50 into and through the tubular filter 50 (which is known as outside-in flow), the fluid to be filtered (illustrated by arrows in phantom) passes into and begins to flow through the filter media 48. The fluid in the filter media 48 flows longitudinally along the filter wall 70, as shown by arrows 78, toward the point at which the tubular filter 50 and the outlet end 44 of the housing member 40 converge. After the fluid flows longitudinally for a given period of time, the fluid flow then moves laterally, as shown by arrows 76, into the filter cavity 72 where it can exit from the filter assembly 10 through the opening 45 in the outlet end 44. The period of longitudinal flow, as shown by the arrows 78, is of sufficient time to wash the filter wall 70 and the closed end 52 of the tubular filter 50 of impurities and to continue the flow of any impurities along the filter 50 as the fluid to be filtered passes into and through the tubular filter 50. The washed surfaces thereby enable fluid to be filtered to contact a clean surface, essentially free of impurities. Thus, the fluid which has been filtered and has entered into the filter cavity 72 is essentially free from impurities or the like, and fluid that continues to enter into the tubular filter will be free of impurities since the impurities have been washed toward and collected at the molded, secured end 54 of the tubular filter 50.

Referring to Figure 4, an alternative embodiment of the present invention is shown. In the alternative embodiment, the features that are the same as in the preferred embodiment are identified with the same numerals having a prime, and the features which are new are identified as such.

The filter assembly 10' is essentially identical to that previously described. The elongated hollow tubular housing member 40' includes an outlet end 44' and an inlet end 42' to allow attachment of the housing member 40' to a needle 13' and fluid source 12', respectively. The tubular filter 50' is substantially identical to the tubular filter of the preferred embodiment and is secured at the outlet end 44' of the housing member 40'. As previously described, the tubular filter 50' defines a cavity 72' therein.

A support member 62 is positioned within the filter cavity 72'. The support member 62 generally maintains the shape of the tubular filter 50' and prevents the filter 50' from collapsing during filtration without impeding the flow of the fluid therein. The support member 62 can have a generally cylindrical core member 63 extending into the filter cavity 72' and fixed at its other end at the outlet end 44' of the housing member 40'. Alternatively, the support member 62 can include a mesh-like matrix disposed on either the inside or the outside of the tubular filter (not shown). The support member 62 can be constructed of any suitable material, preferably the support member 62 is constructed of the same material as comprises the housing member 40'.

The present invention further provides a method of making the filter assembly 10,10' by injection molding a housing member 40,40' having the outlet end 44,44' and the inlet end 42,42' while simultaneously sealing the outlet end 44,44' of the housing member 40,40' to the open end 54,54' of the tubular filter media 50,50'. That is, a one-piece, unitary housing 40,40' is formed while simultaneously sealing the open end 54,54' of the tubular filter 50,50' to the outlet end 44,44' of the housing member 40,40'. The injection molding is accomplished by techniques well known to those skilled in the art.

The present invention can be practiced with various shaped filter medias as long as the media can be supported prior to molding, and the peripheral edge of the media is available for bonding to the housing outlet during the one step molding process.

In the preferred embodiment, in order to simultaneously form the housing member 40,40' and seal the tubular filter 50,50' within the housing member 40,40', the tubular filter media 48 is placed onto a mandrel prior to the injection molding of the housing member 40,40'. The mandrel having the filter media 48 thereon is inserted into a die. The die can consist of a standard two piece die defining a cavity which is well known in the art. After inserting the mandrel including the filter media 48 thereon, a plastic material is injected into the mold therein filling the cavity defined by the die halves and forming the housing member 40,40'. During the injection step, the plastic material injected into the die fills the cavities of the die and surrounds the mandrel. As the plastic surrounds the mandrel, the opening 45,45' in the outlet end 44,44' and the passageway 46,46' the housing member 40,40' are formed.

Additionally, during formation of the opening 45,45' in the outlet end 44,44', the peripheral edge 68,68' of the tubular filter 50,50' is simultaneously sealed to the outlet end 44,44' of the housing member 40,40'. The open end 54,54' of the tubular filter member 50,50' is simultaneously molded to the inner surface 66,66' of the outlet end 44,44' of the housing member 40,40' thereby creating a fluid-tight seal between the filter member 50,50' and the housing member 40,40'. Both the internal and external tapers on the housing member 40,40' can be simultaneously formed during the injection molding process.

The length of the tubular filter member 50,50' can be varied from extending approximately one quarter of the length to three-quarters of the length of the internal passageway 46,46' in order to increase or decrease the amount of filter surface area available for filtering.

The method of forming the filter assembly 10,10' can also include the step of disposing a support member 62 within the housing member 40,40' for retaining the shape of the filter 50,50' during filtering.

The invention has been described in an illustrative manner, and it is to be understood the terminology used is intended to be in the nature of description rather than of limitation.

Obviously, many modifications and variations of the present invention are possible in light of the above teachings. Therefore, it is to be understood that within the scope of the appended claims, reference numerals are merely for convenience and are not to be in any way limiting, the invention may be practiced otherwise than as specifically described.

## Claims

1. A method of making a filter assembly (10,10') by injection molding a housing member (40,40') having an outlet end (44,44') and an inlet end (42,42') while simultaneously sealing the outlet end (44,44') of the housing (40,40') to a peripheral edge (68,68') of a filter media (48,48').

2. A method as set forth in claim 1, wherein the filter media (48,48') is tubular in shape includes an open end (54,54') and a closed end (52,52') extending into the housing (40,40').

3. A method as set forth in claim 2, further including the step of placing a tubular filter media (50,50') on a mandrel prior to injection molding of the housing member (40,40').

4. A method as set forth in claim 2, wherein said sealing step is further defined as sealing an inner surface (66,66') of the outlet end (44,44') of the housing member (40,40') to the open end (54,54') of the tubular filter media (50,50').

5. A method as set forth in claim 2, wherein said sealing step is further defined as sealing the peripheral edge (68,68') of the open end (54,54') of the tubular filter media (50,50') to the housing member (40,40').

6. A method as set forth in claim 1 further including the step of tapering an external surface (56,56') of the housing member (40,40') whereby the external surface (56,56') narrows toward the outlet end (44,44').

7. A method as set forth in claim 1 further including the step of tapering an internal surface (64,64') of the housing member (40,40'), whereby the internal surface (64,64') narrows toward the outlet end (44,44').

8. A method according to claim 1 further including the step of disposing a connector (58,58') at the inlet end (42,42') of the housing member (40,40') for connecting the filter assembly (10,10') to a fluid source.

9. A method according to claim 1 further including the step of disposing a support (62) within the housing member (40') for retaining the shape of the filter (48') during filtering, the support (62) allowing fluid to pass therethrough and exit the filter assembly (10').

10. A filter assembly (10,10') comprising:
housing means (40,40') having an inlet end (42,42') and an outlet end (44,44') and an internal passageway (46,46') therethrough for allowing fluid flow therethrough, said outlet end (44,44'), being smaller in diameter than the remainder of said passageway (46,46'); and
filter media (48,48') defining a tube (50,50') having a closed end (52,52') and an open end (54,54'), said open end (54,54') being sealed to said outlet end (44,44') and the remainder of said tube (50,50') extending into said passageway (46,46').

11. A filter assembly (10,10') according to claim 10, wherein said housing means (40,40') consists of a single piece housing.

12. A filter assembly (10,10') according to claim 10, wherein said housing (40,40'), includes an external surface (56,56'), said external surface (56,56') being tapered, said tapered external surface (56,56') narrowing towards said outlet end (44,44').

13. A filter assembly (10,10') according to claim 10, wherein said housing (40,40') includes connector means (58,58') disposed at said inlet end (42,42') for connecting said filter assembly (10,10') to a fluid source.

14. A filter assembly (10') according to claim 10 including support means (62) disposed within said housing (40') for retaining the shape of said filter media (48') during filtering, said support means (62) allowing fluid to pass therethrough and exit said filter assembly (10').

15. A filter assembly (10') according to claim 14, wherein said support means (62) includes mesh disposed on the inside or outside of said tube (50').

16. A filter assembly 10,10') according to claim 10, wherein said outlet end (44,44') includes an inner surface 66,66'), said open end (54,54') of said tube (50,50') being sealed to said inner surface (66,66') of said outlet end (44,44').

17. A filter assembly (10,10') according to claim 10, wherein said housing (40,40') includes an internal surface (64,64), said internal surface (64,64') being tapered, said tapered internal surface (64,64') narrowing towards said outlet end (44,44').
